# EUROPEAN PATENT SPECIFICATION

(11) **EP 0 719 339 B1**
(45) Date of publication and mention of the grant of the patent: **25.10.2006**
(21) Application number: 94929758.4
(22) Date of filing: 31.08.1994
(51) Int. Cl.: C12N 15/57, C11D 3/386, C12N 9/54

(54) **SUBTILISIN BPN' VARIANTS WITH DECREASED ADSORPTION AND INCREASED HYDROLYSIS**
SUBTILISIN BPN'-VARIANTEN MIT VERMINDERTER ADSORPTION UND ERHÖHTER HYDROLYSE
VARIANTES DE BPN' DE LA SUBTILYSINE PRESENTANT UNE ADSORPTION REDUITE ET UNE MEILLEURE HYDROLYSE

(30) Priority: 15.09.1993 US 121437; 11.08.1994 US 287461
(43) Date of publication of application: 03.07.1996
(73) Proprietor: THE PROCTER & GAMBLE COMPANY, Cincinnati, Ohio 45202 (US)
(72) Inventor: BRODE, Philip, Frederick, III, Cincinnati, OH 45252 (US); BARNETT, Bobby, Lee, Cincinnati, OH 45240 (US); RUBINGH, Donn, Nelton, Cincinnati, OH 45247 (US)
(74) Representative: Kellenberger, Jakob
(86) International application number: PCT/US1994/010020
(87) International publication number: WO 1995/007991

(56) References cited:
- EP-A- 0 405 901
- WO-A-89/09819
- WO-A-92/11357
- CHEMICAL ABSTRACTS, vol. 116, no. 23, 8 June 1992, Columbus, Ohio, US; abstract no. 230623, P. BRODE AND D. RAUCH 'Subtilisin BPN': activity on an immobilized substrate' & LANGMUIR, vol.8, no.5, 1992 pages 1325 - 1329 cited in the application

## Description

### TECHNICAL FIELD

The present invention relates to novel enzyme variants useful in a variety of cleaning compositions, and such cleaning compositions.

### BACKGROUND

Enzymes make up the largest class of naturally occurring proteins. Each class of enzyme generally catalyzes (accelerates a reaction without being consumed) a different kind of chemical reaction. One class of enzymes known as proteases, are known for their ability to hydrolyze (break down a compound into two or more simpler compounds with the uptake of the H and OH parts of a water molecule on either side of the chemical bond cleaved) other proteins. This ability to hydrolyze proteins has been taken advantage of by incorporating naturally occurring and protein engineered proteases as an additive to laundry detergent preparations. Many stains on clothes are proteinaceous and wide-specificity proteases can substantially improve removal of such stains.

Unfortunately, the efficacy level of these proteins in their natural, bacterial environment, frequently does not translate into the relatively unnatural wash environment. Specifically, protease characteristics such as thermal stability, pH stability, oxidative stability and substrate specificity are not necessarily optimized for utilization outside the natural environment of the enzyme.

The amino acid sequence of the protease determines the characteristics of the protease. A change of the amino acid sequence of the protease may alter the properties of the enzyme to varying degrees, or may even inactivate the enzyme, depending upon the location, nature and/or magnitude of the change in the amino acid sequence. Several approaches have been taken to alter the wild-type amino acid sequence of proteases in an attempt to improve their properties, with the goal of increasing the efficacy of the protease in the wash environment. These approaches include altering the amino acid sequence to enhance thermal stability and to improve oxidation stability under quite diverse conditions.

Despite the variety of approaches described in the art, there is a continuing need for new effective variants of proteases useful for cleaning a variety of surfaces.

### Obiects of the Present Invention

It is an object of the present invention to provide subtilisin enzyme variants having improved hydrolysis versus the wild-type of the enzyme.

It is also an object of the present invention to provide cleaning compositions comprising these subtilisin enzyme variants.

### SUMMARY

The present invention relates to a subtilisin BPN' variant comprising wild-type amino acid sequence, characterized in that the wild-type amino acid sequence comprises one of the following specific mutations, wherein the original amino acid occurring in wild-type is given first, the position number second, and the substituted amino acid third:

### - single mutation:

Lys213Glu:
Ala216Glu;
Ala216Asp;
Ala216Gly;
Ser204Glu;
Val203Glu;

### - double mutation:

Lys213Glu + Tyr217Leu;
Ile205Leu + Ala216Glu;
Ile205Leu + Ala216Asp;
Pro210Ala + Gly215Thr;
Lys213Glu + Ala216Glu;
Tyr214Phe + Tyr217Asn;
Gln206Glu + Ala216Glu;
Ala216Glu + Tyr217Leu;
Gln206Glu + Tyr217Leu;
Gln206Glu + Lys213Glu;

### - triple mutation:

Gln206Pro + Gly211Ala + Ala216Glu;
Lys213Glu + Ala216glu + Tyr217Leu;
Ile205Val + Pro210Ala + Lys213Glu;
Gln206Glu + Ala216Glu + Tyr217Leu;
Gln206Glu + Lys213Glu + Tyr217Leu;

### - quadruple mutation:

Pro210Ala + Lys213Glu + Ala216Glu + Tyr217 Leu;
Gln206Glu + Lys213Glu + Ala216Glu + Tyr217Leu;
Ser204Glu + Gln206Glu + Ala216Glu + Tyr217 Leu;

### - quintuple mutation:

Ile205Leu + Pro210Ala + Lys213Glu + Ala216Glu + Tyr217Leu; and
Ser204Glu + Gln206Glu + Lys213Glu + Ala216Glu + Tyr217 Leu,
characterized in that the BPN' variants have decreased adsorption to and increased hydrolysis of, an insoluble substrate as compared to wild-type Subtilisin BPN'. The present invention also relates to cleaning compositiona selected from the group consisting of a hard surface cleaning composition, a dishwashing composition, an oral cleaning composition, a denture cleansing composition and a contact lens cleaning composition, characterized in that the cleaning composition comprises said BPN' variant and a hard surface cleaning carrier.

### DESCRIPTION

### I. Subtilisin Variants

This invention pertains to subtilisin enzymes, in particular BPN', that have been modified by mutating the various nucleotide sequences that code for the enzyme, thereby modifying the amino acid sequence of the enzyme. The modified subtilisin enzymes (hereinafter, "BPN' variants") of the present invention have decreased adsorption to and increased hydrolysis of an insoluble substrate as compared to the wild-type subtilisin. The present invention also pertains to the mutant genes encoding for such BPN' variants.

The subtilisin enzymes of this invention belong to a class of enzymes known as proteases. A protease is a catalyst for the cleavage of peptide bonds. One type of protease is a serine protease. A serine protease is distinguished by the fact that there is an essential serine residue at the active site.

The observation that an enzyme's rate of hydrolysis of soluble substrates increases with enzyme concentration is well documented. It would therefore seem plausible that for surface bound substrates, such as is encountered in many cleaning applications, the rate of hydrolysis would increase with increasing surface concentration. This has been shown to be the case. (Brode, P.F. III and D. S. Rauch, LANGMUIR, "Subtilisin BPN': Activity on an Immolbilized Substrate", Vol. 8, pp. 1325-1329 (1992)). In fact, a linear dependence of rate upon surface concentration was found for insoluble substrates when the surface concentration of the enzyme was varied. (Rubingh, D. N. and M. D. Bauer, "Catalysis of Hydrolysis by Proteases at the Protein-Solution Interface," in POLYMER SOLUTIONS. BLENDS AND INTERFACES, Ed. by I. Noda and D. N. Rubingh, Elsevier, p. 464 (1992)). Surprisingly, when seeking to apply this principle in the search for variant proteases which give better cleaning performance, we did not find that enzymes which adsorb more give better performance. In fact, we surprisingly determined the opposite to be the case: decreased adsorption by an enzyme to a substrate resulted in increased hydrolysis of the substrate (i.e., better cleaning performance).

While not wishing to be bound by theory, it is believed that improved performance, when comparing one variant to another, is a result of the fact that enzymes which adsorb less are also less tightly bound and therefore more highly mobile on the surface from which the insoluble protein substrate is to be removed. At comparable enzyme solution concentrations, this increased mobility is sufficient to outweigh any advantage that is conferred by delivering a higher concentration of enzyme to the surface.

The mutations described herein are designed to change (i.e., decrease) the adsorption of the enzyme to surface-bound soils. In BPN', the amino acids from position 200 to position 220 form a large exterior loop on the enzyme molecule. It has been discovered that this loop plays a significant role in the adsorption of the enzyme molecule to a surface-bound peptide, and specific mutations in this loop have a significant effect on this adsorption. While not wishing to be bound by theory, it is believed that this loop is important to the adsorption of the BPN' molecule for at least two reasons. First, the amino acids which comprise this exterior loop can make close contacts with any surfaces to which the molecule is exposed. Second, the proximity of this loop to the active-site and binding pocket of the BPN' molecule gives it a role in the catalytically productive adsorption of the enzyme to surface-bound substrates (peptides/protein soils).

As used herein, "variant" means an enzyme having an amino acid sequence which differs from that of wild-type.

As used herein, "mutant BPN' gene" means a gene coding for a BPN' variant.

As used herein, "wild-type subtilisin BPN'" refers to a subtilisin enzyme represented by SEQ ID NO:1. The amino acid sequence for subtilitisn BPN' is further described by Wells, J. A., E. Ferrari, D. J. Henner, D. A. Estell and E. Y. Chen, NUCLEIC ACIDS RESEARCH, Vol. II, 7911-7925 (1983).

As used herein, the term "wild-type amino acid sequence" encompasses SEQ ID NO:1 as well as SEQ ID NO:1 having modifications to the amino acid sequence other than at any of positions 199-220.

As used herein, "more hydrophilic amino acid" refers to any other amino acid having greater hydrophilicity than a subject amino acid with reference to the hydrophilicity table below. The following hydrophilicity table (Table 1) lists amino acids in descending order of increasing hydrophilicity (see Hopp, T.P., and Woods, K.R., "Prediction of Protein Antigenic Determinants from Amino Acid Sequences", PROCEEDINGS OF THE NATIONAL ACADEMY OF SCIENCE USA, Vol. 78, pp. 3824-3828, 1981, incorporated herein by reference).

**TABLE 1**

| Amino Acid | Hydrophilicity Value |
|---|---|
| Trp | -3.4 |
| Phe | -2.5 |
| Tyr | -2.3 |
| Leu, Ile | -1.8 |
| Val | -1.5 |
| Met | -1.3 |
| Cys | -1.0 |
| Ala, His | -0.5 |
| Thr | -0.4 |
| Pro, Gly | -0.0 |
| Gln, Asn | 0.2 |
| Ser | 0.3 |
| Arg⁺, Lys⁺, Glu-, Asp- | 3.0 |

Table 1 also indicates which amino acids carry a charge (this characteristic being based on a pH of from about 8-9). The positively charged amino acids are Arg and Lys, the negatively charged amino acids are Glu and Asp, and the remaining amino acids are neutral. In a preferred embodiment of the present invention, the substituting amino acid is either neutral or negatively charged, more preferably negatively charged (i.e., Glu or Asp).

### A. Preparation of enzyme variants

### Example 1

### Mutant BPN' Genes

A phagemid (pSS-5) containing the wild type subtilisin BPN' gene (Mitchinson, C. and J. A. Wells, (1989), "Protein Engineering of Disulfide Bonds in Subtilisin BPN', BIOCHEMISTRY, Vol. 28, pp. 4807-4815) is transformed into *Escherichia coli ung*-strain CJ236 and a single stranded uracil-containing DNA template is produced using the VCSM13 helper phage (Kunkel, T.A., J.D. Roberts and R.A. Zakour, "Rapid and efficient site-specific mutagenesis without phenotypic selection", METHODS IN ENZYMOLOGY, Vol. 154, pp. 367-382, (1987); as modified by Yuckenberg, P.D., F. Witney, J. Geisselsoder and J. McClary, "Site-directed in vitro mutagenesis using uracil-containing DNA and phagemid vectors", DIRECTED MUTAGENESIS - A PRACTICAL APPROACH, ed. M.J. McPherson, pp. 27-48, (1991).

A single primer site-directed mutagenesis modification of the method of Zoller and Smith (Zoller, M.J., and M. Smith, "Oligonucleotide-directed mutagenesis using M13-derived vectors: an efficient and general procedure for the production of point mutations in any fragment of DNA", NUCLEIC ACIDS RESEARCH, Vol. 10, pp. 6487-6500, (1982)) is used to produce all mutants (basically as presented by Yuckenberg, *et al.,* 1991, above). Oligonucleotides are made using an Applied Biosystem Inc. 380B DNA synthesizer. Mutagenesis reaction products are transformed into *Escherichia coli* strain MM294 (American Type Culture Collection *E. Coli.* 33625). All mutants are confirmed by DNA sequencing and the isolated DNA is transformed into the *Bacillus subtilis* expression strain BG2036 (Yang, M. Y., E. Ferrari and D. J. Henner, (1984), "Cloning of the Neutral Protease Gene of *Bacillus subtillis* and the Use of the Cloned Gene to Create an *In Vitro*-derived Deletion Mutation", JOURNAL OF BACTERIOLOGY, Vol. 160, pp. 15-21). For some of the mutants a modified pSS-5 with a frameshift-stop codon mutation at amino acid 217 is used to produce the uracil template. Oligonucleotides are designed to restore the proper reading frame at position 217 and also encoded for random substitutions at positions 199, 200, 201, 202, 203, 204, 205, 206, 207, 208, 209, 210, 211, 212, 213, 214, 215, 216, 217, 218, 219 and 220 (equimolar and/or variable mixtures of all four nucleotides for all three bases at these codons). Mutations that correct for the frameshift-stop and produce a functional enzyme are identified by their ability to digest casein. The random substitutions are determined by DNA sequencing.

### Example 2

### Fermentation

The *Bacillus subtilis* cells (BE2036) containing a subtilisin mutant of interest are grown to mid-log phase in a one liter culture of LB-glucose broth and inoculated into a Biostat ED fermenter (B. Braun Biotech, Inc., Allentown, Pennsylvania) in a total volume of 10 liters. The fermentation media contains Yeast Extract, starch, antifoam, buffers and trace minerals (see FERMENTATION: A PRACTICAL APPROACH, Ed. B. McNeil and L. M. Harvey, 1990). The broth is kept at a constant pH of 7.0 during the fermentation run. Chloramphenical is added for antibiotic selection of mutagenized plasmid. The cells are grown overnight at 37°C to an A₆₀₀ of about 60 and harvested.

### Example 3

### Purification

The fermentation broth is taken through the following steps to obtain pure enzyme. The broth is cleared of *Bacillus subtilis* cells by centrifugation, and clarified by removing fine particulates with a 100K cutoff membrane. This is followed by concentration on a 10K cutoff membrane, and flow dialysis to reduce the ionic strength and adjust the pH to 5.5 using 0.025M MES buffer (2-(*N*-morpholino)ethanesulfonic acid). The enzyme is further purified by loading it onto either a cation exchange chromatography column or an affinity adsorption chromatography column and eluting it from the column with a NaCl or a propylene glycol gradient (see Scopes, R. K., PROTEIN PURIFICATION PRINCIPLES AND PRACTICE, Springer-Verlag, New York (1984)).

The *p*NA assay (DelMar, E.G., C. Largman, J.W. Brodrick and M.C. Geokas, ANAL. BIOCHEM., Vol. 99, pp. 316-320, (1979)) is used to determine the active enzyme concentration for fractions collected during gradient elution. This assay measures the rate at which p-nitroaniline is released as the enzyme hydrolyzes the soluble synthetic substrate, succinyl-alanine-alanine-proline-phenylalanine-*p*-nitroanilide (sAAPF-*p*NA). The rate of production of yellow color from the hydrolysis reaction is measured at 410 nm on a spectrophotometer and is proportional to the active enzyme concentration. In addition, absorbance measurements at 280 nm are used to determine the total protein concentration. The active enzyme/total-protein ratio gives the enzyme purity, and is used to identify fractions to be pooled for the stock solution.

To avoid autolysis' of the enzyme during storage, an equal weight of propylene glycol is added to the pooled fractions obtained from the chromatography column. Upon completion of the purification procedure the purity of the stock enzyme solution is checked with SDS-PAGE (sodium dodecyl sulfate polyacrylamide gel electrophoresis) and the absolute enzyme concentration is determined via an active site titration method using trypsin inhibitor type II-T: turkey egg white purchased from Sigma Chemical Company (St. Louis, Missouri). The measured conversion factors will show which changes made in the enzyme molecule at the various positions result in the enzyme variant having increased activity over the wild-type, against the soluble substrate *p*NA.

In preparation for use, the enzyme stock solution is eluted through a Sephadex-G25 (Pharmacia, Piscataway, New Jersey) size exclusion column to remove the propylene glycol and exchange the buffer. The MES buffer in the enzyme stock solution is exchanged for 0.1 M Tris buffer (Tris(hydroxymethylaminomethane) containing 0.01 M CaCl₂ and pH adjusted to 8.6 with HCl. All experiments are carried out at pH 8.6 in Tris buffer thermostated at 25°C.

### B. Characterization of enzyme variants

### Example 4

### Model Surface Preparation

Aminopropyl controlled pore glass (CPG) purchased from CPG Inc. (Fairfield, New Jersey) is used as a support for covalently attaching the sAAPF-*p*NA substrate purchased from Bachem, Inc. (Torrence, Califomia). The reaction is carried out in dimethyl sulfoxide and (1-ethyl-3-[3-(dirnethylamino)propyl] carbodiimide hydrochloride) (EDC) is used as a coupling agent. Upon completion (monitored by pNA assay), the excess solvent is removed, and the CPG:sAAPF-*p*NA is rinsed with dimethyl sulfoxide (DMSO) and doubly-distilled water. This is followed by oven drying with a N₂ purge at about 70°C. The reaction scheme and preparation of the immobilized substrate are conducted as described by Brode, P.F. III, and D.S. Rauch, "Subtilisin BPN': Activity on an Immobilized Substrate," LANGMUIR, Vol. 8, p. 1325-1329, (1992).

The CPG surface will have 62,000 ± 7,000 *p*NA molecules/µm². The surface area will remain unchanged from the value of 50.0m²/g reported by CPG Inc. for the CPG as received. This suggests that the procedure used to add sAAPF-*p*NA to CPG does not damage the porous structure (mean diameter is 486 A).

### Example 5

### Surface Hydrolysis Assay

Using CPG:sAAPF-*p*NA, adsorption of an enzyme variant and hydrolysis of a CPG-bound peptide can be measured in a single experiment. A small volume of enzyme variant stock solution is added to a flask containing Tris buffer and CPG:sAAPF-*p*NA which has been degassed. The flask is shaken on a wrist-action shaker for a period of 90 minutes during which the shaker is stopped at various time intervals (for example, every 2 minutes during the early stages of adsorption hydrolysis - e.g., the first 20 minutes - and every 10 minutes towards the end of the experiment). The CPG:sAAPF-*p*NA is allowed to settle and the solution is sampled. Both the experimental procedure and the calculation of the adsorption and hydrolysis are conducted as described by Brode *et al.,* 1992, above.

All enzymes are monitored for stability against autolysis and should show no appreciable autolytic loss over the time course of this experiment. Therefore, enzyme adsorption can be determined by measuring solution depletion. The difference between the initial enzyme variant concentration and the concentration measured at each individual time point gives the amount of enzyme variant adsorbed. The amount of *p*NA hydrolyzed from the surface is measured by taking an absorbance reading on an aliquot of the sample at 410 nm. The total amount of *p*NA hydrolyzed is calculated by adding the amount sampled and the amount remaining in the flask. This value is corrected by subtracting the amount of *p*NA that is hydrolyzed by Tris buffer at pH 8.6 when no enzyme is present. This base-hydrolysis ranges from 7-29% of the total hydrolysis depending on the efficiency of the enzyme.

### Example 6

### Soluble Substrate Kinetic Analysis

The rates of hydrolysis of the soluble substrate sAAPF-*p*NA are monitored by measuring the adsorbance increase as a function of time at 410 nm on a DU-70 spectrophotometer. The enzyme concentration is held constant and is prepared to be in the range of 6-10 nanomolar while the substrate concentration is varied from 90-700 µM sAAPF-*p*NA for each kinetic determination. An adsorbance data point is taken each second over a period of 900 seconds and the data are transferred to a LOTUS^{™} spreadsheet (Lotus Development Corporation, Cambridge, Massachusetts). Analysis for kinetic parameters is conducted by the standard Lineweaver Burk analysis in which the data in the initial part of the run (generally the first minute) are fit to a linear regression curve to give vₒ. The vₒ and sₒ data are plotted in the standard inverse fashion to give K_{M} and k_{cat}.

### C. Example BPN' variants

BPN' variants of the present invention which have decreased adsorption to and increased hydrolysis of surface bound substrates are exemplified in Table 2, below. In describing the specific mutations, the original amino acid occurring in wild-type is given first, the position number second, and the substituted amino acid third.

**TABLE 2**

| Example BPN' Variants |
|---|
| -Single Mutation- |
| Lys213Glu |
| Ala216Glu |
| Ala216Asp |
| Ala216Gly |
| Ser204Glu |
| Val203Glu |

| -Double Mutation- |
|---|
| Lys213Glu + Tyr217Leu |
| Ile205Leu + Ala216Glu |
| Ile205Leu + Ala21.6Asp |
| Pro210Ala + Gly215Thr |
| Lys213Glu + Ala216Glu |
| Tyr214Phe + Tyr217Asn |
| Gln206Glu + Ala216Glu |
| Ala216Glu + Tyr217Leu |
| Gln206Glu + Tyr217Leu |
| Gln206Glu + Lys213Glu |

| -Triple Mutation- |
|---|
| Gln206Pro + Gly211Ala + Ala216Glu |
| Lys213Glu + Ala216Glu + Tyr217Leu |
| Ile205Val + Pro210Ala + Lys213Glu |
| Gln206Glu + Ala216Glu + Tyr217Leu |
| Gln206Glu + Lys213Glu + Tyr217Leu |

| -Quadruple Mutation- |
|---|
| Pro210Ala + Lys213Glu + Ala216Glu + Tyr217Leu |
| Gln206Glu + Lys213Glu + Ala216Glu + Tyr217Leu |
| Ser204Glu + Gln206Glu + Ala216Glu + Tyr217Leu |

| -Quintuple Mutation- |
|---|
| Ile205Leu + Pro210Ala + Lys213Glu + Ala216Glu + Tyr217Leu |
| Ser204Glu + Gln206Glu + Lys213Glu + Ala216Glu + |
| Tyr217Leu |

### II. Cleaning Compositions

In another embodiment of the present invention, an effective amount of one or more enzyme variants of the present invention are included in compositions useful for cleaning a variety of surfaces in need of proteinaceous stain removal. Such cleaning compositions include detergent compositions for cleaning hard surfaces, unlimited in form (e.g., liquid and granular); detergent compositions for cleaning fabrics, unlimited in form (e.g., granular, liquid and bar formulations); dishwashing compositions (unlimited in form); oral cleaning compositions, unlimited in form (e.g., dentifrice, toothpaste and mouthwash formulations); denture cleaning compositions, unlimited in form (e.g., liquid, tablet); and contact lens cleaning compositions, unlimited in form (e.g., liquid, tablet). As used herein, "effective amount of enzyme variant" refers to the quantity of enzyme variant necessary to achieve the enzymatic activity necessary in the specific cleaning composition. Such effective amounts are readily ascertained by one of ordinary skill in the art and is based on many factors, such as the particular enzyme variant used, the cleaning application, the specific composition of the cleaning composition, and whether a liquid or dry (e.g., granular, bar) composition is required, and the like. Preferably the cleaning compositions of the present invention comprise from about 0.0001 % to about 10% of one or more enzyme variants of the present invention, more preferably from about 0.001% to about 1%, more preferably still from about 0.01% to about 0.1%. Several examples of various cleaning compositions wherein the enzyme variants of the present invention may be employed are discussed in further detail below. All parts, percentages and ratios used herein are by weight unless otherwise specified.

As used herein, "non-fabric cleaning compositions" include hard surface cleaning compositions, dishwashing compositions, oral cleaning compositions, denture cleaning compositions and contact lens cleaning compositions.

### A. Cleaning Compositions for Hard Surfaces, Dishes and Fabrics

The enzymes of the present invention can be used in any detergent composition where high sudsing and good insoluble substrate removal are desired. Thus the enzyme variants of the present invention can be used with various conventional ingredients to provide fully-formulated hard-surface cleaners, dishwashing compositions, fabric laundering compositions and the like. Such compositions can be in the form of liquids, granules, bars and the like. Such compositions can be formulated as modem "concentrated" detergents which contain as much as 30%-60% by weight of surfactants.

The cleaning compositions herein can optionally, and preferably, contain various anionic, nonionic, zwitterionic, etc., surfactants. Such surfactants are typically present at levels of from about 5% to about 35% of the compositions.

Nonlimiting examples of surfactants useful herein include the conventional C₁₁-C₁₈ alkyl benzene sulfonates and primary and random alkyl sulfates, the C₁₀-C₁₈ secondary (2,3) alkyl sulfates of the formulas CH₃(CH₂)x(CHOSO₃)⁻M⁺)CH₃ and CH₃(CH₂)y(CHOSO₃⁻M⁺) CH₂CH₃ wherein x and (y+1) are integers of at least about 7, preferably at least about 9, and M is a water-solubilizing cation, especially sodium, the C₁₀-C₁₈ alkyl alkoxy sulfates (especially EO 1-5 ethoxy sulfates), C₁₀-C₁₈ alkyl alkoxy carboxylates (especially the EO 1-5 ethoxycarboxylates), the C₁₀-C₁₈ alkyl polyglycosides, and their corresponding sulfated polyglycosides, C₁₂-C₁₈ alpha-sulfonated fatty acid esters, C₁₂-C₁₈ alkyl and alkyl phenol alkoxylates (especially ethoxylates and mixed ethoxy/propoxy), C₁₂-C₁₈ betaines and sulfobetaines ("sultaines"), C₁₀-C₁₈ amine oxides, and the like. The alkyl alkoxy sulfates (AES) and alkyl alkoxy carboxylates (AEC) are preferred herein. (Use of such surfactants in combination with the aforesaid amine oxide and/or betaine or sultaine surfactants is also preferred, depending on the desires of the formulator.) Other conventional useful surfactants are listed in standard texts. Particularly useful surfactants include the C₁₀-C₁₈ N-methyl glucamides disclosed in US Patent 5, 194,639, Connor et al., issued March 16, 1993.

A wide variety of other ingredients useful in detergent cleaning compositions can be included in the compositions herein, including other active ingredients, carriers, hydrotropes, processing aids, dyes or pigments, solvents for liquid formulations, etc. If an additional increment of sudsing is desired, suds boosters such as the C₁₀-C₁₆ alkolamides can be incorporated into the compositions, typically at about 1% to about 10% levels. The C₁₀₋C₁₄ monoethanol and diethanol amides illustrate a typical class of such suds boosters. Use of such suds boosters with high sudsing adjunct surfactants such as the amine oxides, betaines and sultaines noted above is also advantageous. If desired, soluble magnesium salts such as MgCl₂, MgSO₄, and the like, can be added at levels of, typically, from about 0.1 % to about 2%, to provide additionally sudsing.

The liquid detergent compositions herein can contain water and other solvents as carriers. Low molecular weight primary or secondary alcohols exemplified by methanol, ethanol, propanol, and isopropanol are suitable. Monohydric alcohols are preferred for solubilizing surfactants, but polyols such as those containing from about 2 to about 6 carbon atoms and from about 2 to about 6 hydroxy groups (e.g., 1,3-propanediol, ethylene glycol, glycerine, and 1,2-propanediol) can also be used. The compositions may contain from about 5% to about 90%, typically from about 10% to about 50% of such carriers.

The detergent compositions herein will preferably be formulated such that during use in aqueous cleaning operations, the wash water will have a pH between about 6.8 and about 11.0. Finished products thus are typically formulated at this range. Techniques for controlling pH at recommended usage levels include the use of buffers, alkalis, acids, etc., and are well known to those skilled in the art.

When formulating, the hard surface cleaning compositions and fabric cleaning compositions of the present invention, the formulator may wish to employ various builders at levels from about 5% to about 50% by weight. Typical builders include the 1-10 micron zeolites, polycarboxylates such as citrate and oxydisuccinates, layered silicates, phosphates, and the like. Other conventional builders are listed in standard formularies.

Likewise, the formulator may wish to employ various additional enzymes, such as cellulases, lipases, amylases and proteases in such compositions, typically at levels of from about 0.001% to about 1% by weight. Various detersive and fabric care enzymes are well-known in the laundry detergent art.

Various bleaching compounds, such as the percarbonates, perborates and the like, can be used in such compositions, typically at levels from about 1% to about 15% by weight. If desired, such compositions can also contain bleach activators such as tetraacetyl ethylenediamine, nonanoyloxybenzene sulfonate, and the like, which are also known in the art. Usage levels typically range from about 1 % to about 10% by weight.

Various soil release agents, especially of the anionic oligoester type, various chelating agents, especially the aminophosphonates and ethylenediaminedisuccinates, various clay soil removal agents, especially ethoxylated tetraethylene pentamine, various dispersing agents, especially polyacrylates and polyasparatates, various brighteners, especially anionic brighteners, various suds suppressors, especially silicones and secondary alcohols, various fabric softeners, especially smectite clays, and the like can all be used in such compositions at levels ranging from about 1% to about 35% by weight. Standard formularies and published patents contain multiple, detailed descriptions of such conventional materials.

Enzyme stabilizers may also be used in the cleaning compositions of the present invention. Such enzyme stabilizers include propylene glycol (preferably from about 1% to about 10%), sodium formate (preferably from about 0.1% to about 1 %) and calcium formate (preferably from about 0.1 % to about 1%).

### 1. Hard surface cleaning compositions

As used herein "hard surface cleaning composition" refers to liquid and granular detergent compositions for cleaning hard surfaces such as floors, walls, bathroom tile, and the like. Hard surface cleaning compositions of the present invention comprise an effective amount of one or more enzyme variants of the present invention, preferably from about 0.001% to about 10%, more preferably from about .01% to about 5%, more preferably still from about .05% to about 1% by weight of active enzyme of the composition. In addition to comprising one or more enzyme variants of the present invention, such hard surface cleaning compositions typically comprise a surfactant and a water-soluble sequestering builder. In certain specialized products such as spray window cleaners, however, the surfactants are sometimes not used since they may produce a filmy/streaky residue on the glass surface.

The surfactant component, when present, may comprise as little as 0.1% of the compositions herein, but typically the compositions will contain from about 0.25% to about 10%, more preferably from about 1% to about 5% of surfactant.

Typically the compositions will contain from about 0.5% to about 50% of a detergency builder, preferably from about 1% to about 10%.

Preferably the pH should be in the range of about 8 to 12. Conventional pH adjustment agents such as sodium hydroxide, sodium carbonate or hydrochloric acid can be used if adjustment is necessary.

Solvents may be included in the compositions. Useful solvents include, but are not limited to, glycol ethers such as diethyleneglycol monohexyl ether, diethyleneglycol monobutyl ether, ethyleneglycol monobutyl ether, ethyleneglycol monohexyl ether, propyleneglycol monobutyl ether, dipropyleneglycol monobutyl ether, and diols such as 2,2,4-trimethyl-1,3-pentanediol and 2-ethyl-1,3-hexanediol. When used, such solvents are typically present at levels of from about 0.5% to about 15%, preferably from about 3% to about 11%.

Additionally, highly volatile solvents such as isopropanol or ethanol can be used in the present compositions to facilitate faster evaporation of the composition from surfaces when the surface is not rinsed after "full strength" application of the composition to the surface. When used, volatile solvents are typically present at levels of from about 2% to about 12% in the compositions.

The hard surface cleaning composition embodiment of the present invention is illustrated by the following examples.

**Examples 7-12**

| Liquid | Hard Surface Cleaning Compositions | | | | | |
|---|---|---|---|---|---|---|
| | Example No. | | | | | |
| Component | 7 | 8 | 9 | 10 | 11 | 12 |
| Lys2l3Glu | 0.05 | 0.50 | 0.02 | 0.03 | 0.10 | 0.03 |
| Ile205Leu + Ala216Asp | - | - | - | - | 0.20 | 0.02 |
| Na₂DIDA* | | | | | | |
| EDTA** | - | - | 2.90 | 2.90 | - | - |
| Na Citrate | - | - | - | - | 2.90 | 2.90 |
| NaC₁₂ Alkyl-benzene sulfonate | 1.95 | - | 1.95 | - | 1.95 | - |
| NaC₁₂ Alkylsulfate | - | 2.20 | - | 2.20 | - | 2.20 |
| NaC₁₂(ethoxy)*** sulfate | - | 2.20 | - | 2.20 | - | 2.20 |
| C₁₂ Dimethylamine oxide | - | 0.50 | - | 0.50 | - | 0.50 |
| Na Cumene sulfonate | 1.30 | - | 1.30 | - | 1.30 | - |
| Hexyl Carbitol*** | 6.30 | 6.30 | 6.30 | 6.30 | 6.30 | 6.30 |
| Water**** | balance to 100% | | | | | |

| | | | | | | |
|---|---|---|---|---|---|---|
| *Disodium N-diethyleneglycol-N,N-iminodiacetate **Na₄ ethylenediamine diacetic acid ***Diethyleneglycol monohexyl ether ****All formulas adjusted to pH 7 | | | | | | |

In Examples 7-10, the BPN' variants recited in Table 2, among others, are substituted for Lys213Glu, with substantially similar results.

In Examples 11-12, any combination of the BPN' variants recited in Table 2, among others, are substituted for Lys213Glu and Ile205Leu + Ala216Asp, with substantially similar results.

**Examples 13-18**

| Spray Compositions for Cleaning Hard Surfaces and Removing Household Mildew | | | | | | |
|---|---|---|---|---|---|---|
| | Example No. | | | | | |
| Component | 13 | 14 | 15 | 16 | 17 | 18 |
| Lys213Glu + Tyr217Leu | 0.50 | 0.05 | 0.60 | 0.30 | 0.20 | 0.30 |
| Ala216Glu | - | - | - | - | 0.30 | 0.10 |
| Sodium octyl sulfate | 2.00 | 2.00 | 2.00 | 2.00 | 2.00 | 2.00 |
| Sodium dodecyl sulfate | 4.00 | 4.00 | 4.00 | 4.00 | 4.00 | 4.00 |
| Sodium hydroxide | 0.80 | 0.80 | 0.80 | 0.80 | 0.80 | 0.80 |
| Silicate (Na) | 0.04 | 0.04 | 0.04 | 0.04 | 0.04 | 0.04 |
| Perfume | 0.35 | 0.35 | 0.35 | 0.35 | .0.35 | 0.35 |
| Water | balance to 100% | | | | | |

### Product pH is about 7.

In Examples 13-16, the BPN' variants recited in Table 2, among others, are substituted for Lys213Glu + Tyr217Leu, with substantially similar results.

In Examples 17-18, any combination of the BPN' variants recited in Table 2, among others, are substituted for Lys213Glu + Tyr217Leu and Ala216Glu, with substantially similar results.

### 2. Dishwashing Compositions

In another embodiment of the present invention, dishwashing compositions comprise one or more enzyme variants of the present invention. As used herein, "dishwashing composition" refers to all forms for compositions for cleaning dishes, including but not limited to, granular and liquid forms. The dishwashing composition embodiment of the present invention is illustrated by the following examples.

**Examples 19-24**

| Dishwashing Composition | | | | | | |
|---|---|---|---|---|---|---|
| | Example No. | | | | | |
| Component | 19 | 20 | 21 | 22 | 23 | 24 |
| Glu206Pro + Gly211Ala | | | | | | |
| + Ala216Glu | 0.05 | 0.50 | 0.02 | 0.40 | 0.10 | 0.03 |
| Ile205Leu + Ala216Asp | - | - | - | - | 0.40 | 0.02 |
| C₁₂-C₁₄ N-methyl-guucamide | 0.90 | 0.90 | 0.90 | 0.90 | 0.90 | 0.90 |
| C₁₂ ethoxy (1) sulfate | 12.00 | 12.00 | 12.00 | 12.00 | 12.00 | 12.00 |
| 2-methyl undecanoic acid | 4.50 | 4.50 | 4.50 | 4.50 | 4.50 | 4.50 |
| C₁₂ ethoxy (2) carboxylate | 4.50 | 4.50 | 4.50 | 4.50 | 4.50 | 4.50 |
| C₁₂ alcohol ethoxylate (4) | 3.00 | 3.00 | 3.00 | 3.00 | 3.00 | 3.00 |
| C₁₂ amine oxide | 3.00 | 3.00 | 3.00 | 3.00 | 3.00 | 3.00 |
| Sodium cumene sulfonate | 2.00 | 2.00 | 2.00 | 2.00 | 2.00 | 2.00 |
| Ethanol | 4.00 | 4.00 | 4.00 | 4.00 | 4.00 | 4.00 |
| Mg⁺⁺ (as MgCl₂) | 0.20 | 0.20 | 0.20 | 0.20 | 0.20 | 0.20 |
| Ca⁺⁺ (as CaCl₂) | 0.40 | 0.40 | 0.40 | 0.40 | 0.40 | 0.40 |
| Water | balance to 100% | | | | | |

### Product pH is adjusted to 7.

In Examples 19-22, the BPN' variants recited in Table 2, among others, are substituted for Gln206Pro + Gly211Ala + Ala216Glu, with substantially similar results.

In Examples 23-24, any combination of the BPN' variants recited in Table 2, among others, are substituted for Gln206Pro + Gly211Ala + Ala216Glu and Ile205Leu + Ala216Asp, with substantially similar results.

### 3. Fabric cleaning compositions

In another embodiment of the present invention, fabric cleaning compositions comprise one or more enzyme variants of the present invention. As used herein, "fabric cleaning composition" refers to all forms for detergent compositions for cleaning fabrics, including but not limited to, granular, liquid and bar forms.

### a. Granular fabric cleaning compositions

The granular fabric cleaning compositions of the present invention contain an effective amount of one or more enzyme variants of the present invention, preferably from about 0.001% to about 10%, more preferably from about 0.005% to about 5%, more preferably from about 0.01% to about 1% by weight of active enzyme of the composition. In addition to one or more enzyme variants, the granular fabric cleaning compositions typically comprise at least one surfactant, one or more builders, and, in some cases, a bleaching agent.

The granular fabric cleaning composition embodiment of the present invention is illustrated by the following examples.

**Examples 25-28**

| Granular Fabric Cleaning Composition | | | | |
|---|---|---|---|---|
| | Example No. | | | |
| Component | 25 | 26 | 27 | 28 |
| Ala216Asp | 0.10 | 0.20 | 0.03 | 0.05 |
| Ala216Gly | - | - | 0.02 | 0.05 |
| C₁₃ linear alkyl benzene sulfonate | 22.00 | 22.00 | 22.00 | 22.00 |
| Phosphate (as sodium tripolyphosphates) | 23.00 | 23.00 | 23.00 | 23.00 |
| Sodium carbonate | 23.00 | 23.00 | 23.00 | 23.00 |
| Sodium silicate | 14.00 | 14.00 | 14.00 | 14.00 |
| Zeolite | 8.20 | 8.20 | 8.20 | 8.20 |
| Chelant (diethylaenetriamine-pentaacetic acid) | 0.40 | 0.40 | 0.40 | 0.40 |
| Sodium sulfate | 5.50 | 5.50 | 5.50 | 5.50 |
| Water | balance to 100% | | | |

In Examples 25-26, the BPN' variants recited in Table 2, among others, are substituted for Ala216Asp, with substantially similar results.

In Examples 27-28, any combination of the BPN' variants recited in Table 2, among others, are substituted for Ala216Asp and Ala216Gly, with substantially similar results.

**Examples 29-32**

| Granular Fabric Cleaning Composition | | | | |
|---|---|---|---|---|
| | Example No. | | | |
| Component | 29 | 30 | 31 | 32 |
| Lys213Glu + Ala216Glu + Tyr217Leu | 0.10 | 0.20 | 0.03 | 0.05 |
| Ile205Val + Pro210Ala + Lys213Glu | - | - | 0.02 | 0.05 |
| C₁₂ alkyl benzene sulfonate | 12.00 | 12.00 | 12.00 | 12.00 |
| Zeolite A (1-10 micrometer) | 26.00 | 26.00 | 26.00 | 26.00 |
| 2-butyl octanoic acid | 4.00 | 4.00 | 4.00 | 4.00 |
| C₁₂-C₁₄ secondary (2,3) alkyl sulfate, Na salt | 5.00 | 5.00 | 5.00 | 5.00 |
| Sodium citrate | 5.00 | 5.00 | 5.00 | 5.00 |
| Optical brightener | 0.10 | 0.10 | 0.10 | 0.10 |
| Sodium sulfate | 17.00 | 17.00 | 17.00 | 17.00 |
| Water and minors | balance to 100% | | | |

In Examples 29-30, the BPN' variants recited in Table 2, among others, are substituted for Lys213Glu + Ala216Glu + Tyr217Leu, with substantially similar results.

In Examples 31-32, any combination of the BPN' variants recited in Table 2, among others, are substituted for Lys213Glu + Ala216Glu + Tyr217Leu and Ile205Val + Pro210Ala + Lys213Glu, with substantially similar results.

### b. Liquid fabric cleaning compositions

Liquid fabric cleaning compositions of the present invention comprise an effective amount of one or more enzyme variants of the present invention, preferably from about 0.005% to about 5%, more preferably from about 0.01 % to about 1 %, by weight of active enzyme of the composition. Such liquid fabric cleaning compositions typically additionally comprise an anionic surfactant, a fatty acid, a water-soluble detergency builder and water.

The liquid fabric cleaning composition embodiment of the present invention is illustrated by the following examples.

**Examples 33-37**

| Liquid Fabric Cleaning Compositions | | | | | |
|---|---|---|---|---|---|
| | Example No. | | | | |
| Component | 33 | 34 | 35 | 36 | 37 |
| Pro210Ala + Gly215Thr | 0.05 | 0.03 | 0.30 | 0.03 | 0.10 |
| Pro210Ala + Lys213Glu + Ala216Glu + Tyr217Leu | - | - | - | 0.01 | 0.20 |
| C₁₂-C₁₄ alkyl sulfate, Na | 20.00 | 20.00 | 20.00 | 20.00 | 20.00 |
| 2-butyl octanoic acid | 5.00 | 5.00 | 5.00 | 5.00 | 5.00 |
| Sodium citrate | 1.00 | 1.00 | 1.00 | 1.00 | 1.00 |
| C₁₀ alcohol ethoxylate (3) | 13.00 | 13.00 | 13.00 | 13.00 | 13.00 |
| Monethanolamine | 2.50 | 2.50 | 2.50 | 2.50 | 2.50 |
| Water/propylene glycol/ethanol | (100:1:1) | balance to 100% | | | |

In Examples 33-35 the BPN' variants recited in Table 2, among others, are substituted for Pro210Ala + Gly215Thr, with substantially similar results.

In Examples 36-37, any combination of the BPN' variants recited in Table 2, among others, are substituted for Pro210Ala + Gly215Thr and Pro210Ala + Lys213Glu + Ala216Glu + Tyr217Leu, with substantially similar results.

### c. Bar fabric cleaning compositions

Bar fabric cleaning compositions of the present invention suitable for hand-washing soiled fabrics contain an effective amount of one or more enzyme variants of the present invention, preferably from about 0.001% to about 10%, more preferably from about 0.01% to about 1% by weight of the composition.

The bar fabric cleaning composition embodiment of the present invention is illustrated by the following examples.

**Examples 38-41**

| Bar Fabric Cleaning Compositions | | | | |
|---|---|---|---|---|
| | Example No. | | | |
| Component | 38 | 39 | 40 | 41 |
| Lys213Glu + Ala216Glu | 0.3 | - | 0.1 | 0.02 |
| Tyr214Phe + Tyr217Asn | - | - | 0.4 | 0.03 |
| C₁₂-C₁₆ alkyl sulfate, Na | 20.0 | 20.0 | 20.0 | 20.00 |
| C₁₂-C₁₄ N-methyl glucamide | 5.0 | 5.0 | 5.0 | 5.00 |
| C₁₁-C₁₃ alkyl benzene sulfonate, Na | 10.0 | 10.0 | 10.0 | 10.00 |
| Sodium carbonate | 25.0 | 25.0 | 25.0 | 25.00 |
| Sodium pyrophosphate | 7.0 | 7.0 | 7.0 | 7.00 |
| Sodium tripolyphosphate | 7.0 | 7.0 | 7.0 | 7.00 |
| Zeolite A (0.1-.10µ) | 5.0 | 5.0 | 5.0 | 5.00 |
| Carboxymethylcellulose | 0.2 | 0.2 | 0.2 | 0.20 |
| Polyacrylate (MW 1400) | 0.2 | 0.2 | 0.2 | 0.20 |
| Coconut monethanolamide | 5.0 | 5.0 | 5.0 | 5.00 |
| Brightener, perfume | 0.2 | 0.2 | 0.2 | 0.20 |
| CaSO₄ | 1.0 | 1.0 | 1.0 | 1.00 |
| MgSO₄ | 1.0 | 1.0 | 1.0 | 1.00 |
| Water | 4.0 | 4.0 | 4.0 | 4.00 |
| Filler* | balance to 100% | | | |

| | | | | |
|---|---|---|---|---|
| *Can be selected from convenient materials such as CaCO₃, talc, clay, silicates, and the like. | | | | |

In Examples 38-39 the BPN' variants recited in Table 2, among others, are substituted for Lys213Glu + Ala216Glu, with substantially similar results.

In Examples 40-41, any combination of the BPN' variants recited in Table 2, among others, are substituted for Lys213Glu + Ala216Glu and Tyr214Phe + Tyr217Asn, with substantially similar results.

### B. Additional Cleaning Compositions

In addition to the hard surface cleaning, dishwashing and fabric cleaning compositions discussed above, one or more enzyme variants of the present invention may be incorporated into a variety of other cleaning compositions where hydrolysis of an insoluble substrate is desired. Such additional cleaning compositions include but are not limited to, oral cleaning compositions, denture cleaning compositions, and contact lens cleaning compositions.

### 1. Oral cleaning compositions

In another embodiment of the present invention, a pharmaceutically-acceptable amount of one or more enzyme variants of the present invention are included in compositions useful for removing proteinaceous stains from teeth or dentures. As used herein, "oral cleaning compositions" refers to dentifrices, toothpastes, toothgels, toothpowders, mouthwashes, mouth sprays, mouth gels, chewing gums, lozenges, sachets, tablets, biogels, prophylaxis pastes, dental treatment solutions, and the like. Preferably, oral cleaning compositions of the present invention comprise from about 0.0001 % to about 20% of one or more enzyme variants of the present invention, more preferably from about 0.001% to about 10%, more preferably still from about 0.01% to about 5%, by weight of the composition, and a pharmaceutically-acceptable carrier. As used herein, "pharmaceutically-acceptable" means that drugs, medicaments or inert ingredients which the term describes are suitable for use in contact with the tissues of humans and lower animals without undue toxicity, incompatibility, instability, irritation, allergic response, and the like, commensurate with a reasonable benefit/risk ratio.

Typically, the pharmaceutically-acceptable oral cleaning carrier components of the oral cleaning components of the oral cleaning compositions will generally comprise from about 50% to about 99.99%, preferably from about 65% to about 99.99%, more preferably from about 65% to about 99%, by weight of the composition.

The pharmaceutically-acceptable carrier components and optional components which may be included in the oral cleaning compositions of the present invention are well known to those skilled in the art. A wide variety of composition types, carrier components and optional components useful in the oral cleaning compositions are disclosed in U.S. Patent 5,096,700, Seibel, issued March 17, 1992; U.S. Patent 5,028,414, Sampathkumar, issued July 2, 1991; and U.S. Patent 5,028,415, Benedict, Bush and Sunberg, issued July 2, 1991;.

The oral cleaning composition embodiment of the present invention is illustrated by the following examples.

**Examples 42-45**

| Dentifrice Composition | | | | |
|---|---|---|---|---|
| | Example No. | | | |
| Component | 42 | 43 | 44 | 45 |
| Ile205Leu + Pro210Ala + Lys213Glu + Ala216Glu + Tyr217Leu | 2.000 | 3.500 | 1.500 | 2.000 |
| Sorbitol (70% aqueous solution) | 35.000 | 35.000 | 35.000 | 35.000 |
| PEG-6* | 1.000 | 1.000 | 1.000 | 1.000 |
| Silica dental abrasive** | 20.000 | 20.000 | 20.000 | 20.000 |
| Sodium fluoride | 0.243 | 0.243 | 0.243 | 0.243 |
| Titanium dioxide | 0.500 | 0.500 | 0.500 | 0.500 |
| Sodium saccharin | 0.286 | 0.286 | 0.286 | 0.286 |
| Sodium alkyl sulfate (27.9% aqueous solution) | 4.000 | 4.000 | 4.000 | 4.000 |
| Flavor | 1.040 | 1.040 | 1.040 | 1.040 |
| Carboxyvinyl Polymer*** | 0.300 | 0.300 | 0.300 | 0.300 |
| Carrageenan**** | 0.800 | 0.800 | 0.800 | 0.800 |
| Water | balance to 100% | | | |

| | | | | |
|---|---|---|---|---|
| *PEG-6 = Polyethylene glycol having a molecular weight of 600. **Precipitated silica identified as Zeodent 119 offered by J.M. Huber. ***Carbopol offered by B.F. Goodrich Chemical Company. ****Iota Carrageenan offered by Hercules Chemical Company. | | | | |

In Examples 42-45 the BPN' variants recited in Table 2, among others, are substituted for Ile205Leu + Pro210Ala + Lys213Glu + Ala216Glu + Tyr217Leu, with substantially similar results.

**Examples 46-49**

| Mouthwash Composition | | | | |
|---|---|---|---|---|
| | Example No. | | | |
| Component | 46 | 47 | 48 | 49 |
| Ala216Gly | 3.00 | 7.50 | 1.00 | 5.00 |
| SDA 40 Alcohol | 8.00 | 8.00 | 8.00 | 8.00 |
| Flavor | 0.08 | 0.08 | 0.08 | 0.08 |
| Emulsifier | 0.08 | 0.08 | 0.08 | 0.08 |
| Sodium Fluoride | 0.05 | 0.05 | 0.05 | 0.05 |
| Glycerin | 10.00 | 10.00 | 10.00 | 10.00 |
| Sweetener | 0.02 | 0.02 | 0.02 | 0.02 |
| Benzoic acid | 0.05 | 0.05 | 0.05 | 0.05 |
| Sodium hydroxide | 0.20 | 0.20 | 0.20 | 0.20 |
| Dye | 0.04 | 0.04 | 0.04 | 0.04 |
| Water | balance to 100% | | | |

In Examples 46-49, the BPN' variants recited in Table 2, among others, are substituted for Ala216Gly, with substantially similar results.

**Examples 50-53**

| Lozenge Composition | | | | |
|---|---|---|---|---|
| | Example No. | | | |
| Component | 50 | 51 | 52 | 53 |
| Tyr214Phe + Tyr217Asn | 0.01 | 0.03 | 0.10 | 0.02 |
| Sorbitol | 17.50 | 17.50 | 17.50 | 17.50 |
| Mannitol | 17.50 | 17.50 | 17.50 | 17.50 |
| Starch | 13.60 | 13.60 | 13.60 | 13.60 |
| Sweetener | 1.20 | 1.20 | 1.20 | 1.20 |
| Flavor | 11.70 | 11.70 | 11.70 | 11.70 |
| Color | 0.10 | 0.10 | 0.10 | 0.10 |
| Corn Syrup | balance to 100% | | | |

In Examples 50-53, the BPN' variants recited in Table 2, among others, are substituted for Tyr214Phe + Tyr217Asn, with substantially similar results.

**Examples 54-57**

| Chewing Gum Composition | | | | |
|---|---|---|---|---|
| | Example No. | | | |
| Component | 54 | 55 | 56 | 57 |
| Ile205Val + Pro210Ala + Lys213Glu | 0.03 | 0.02 | 0.10 | 0.05 |
| Sorbitol crystals | 38.44 | 38.40 | 38.40 | 38.40 |
| Paloja-T gum base* | 20.00 | 20.00 | 20.00 | 20.00 |
| Sorbitol (70% aqueous solution) | 22.00 | 22.00 | 22.00 | 22.00 |
| Mannitol | 10.00 | 10.00 | 10.00 | 10.00 |
| Glycerine | 7.56 | 7.56 | 7.56 | 7.56 |
| Flavor | 1.00 | 1.00 | 1.00 | 1.00 |

| | | | | |
|---|---|---|---|---|
| *Supplied by L.A. Dreyfus Company. | | | | |

In Examples 54-57, the BPN' variants recited in Table 2, among others, are substituted for Ile205Val + Pro210Ala + Lys213Glu, with substantially similar results.

### 2. Denture cleaning compositions

In another embodiment of the present invention, denture cleaning compositions for cleaning dentures outside of the oral cavity comprise one or more enzyme variants of the present invention. Such denture cleaning compositions comprise an effective amount of one or more enzyme variants of the present invention, preferably from about 0.0001% to about 50% of one or more enzyme variants of the present invention, more preferably from about 0.001 % to about 35%, more preferably still from about 0.01 % to about 20%, by weight of the composition, and a denture cleansing carrier. Various denture cleansing composition formats such as effervescent tablets and the like are well known in the art (see for example U.S. Patent 5,055,305, Young), and are generally appropriate for incorporation of one or more enzyme variants of the present invention for removing proteinaceous stains from dentures.

The denture cleaning composition embodiment of the present invention is illustrated by the following examples.

**Examples 58-61**

| Two-layer Effervescent Denture Cleansing Tablet | | | | |
|---|---|---|---|---|
| | Example No. | | | |
| Component | 58 | 59 | 60 | 61 |
| Acidic Layer | | | | |
| Ala216Glu | 1.0 | 1.5 | 0.01 | 0.05 |
| Tartaric acid | 24.0 | 24.0 | 24.00 | 24.00 |
| Sodium carbonate | 4.0 | 4.0 | 4.00 | 4.00 |
| Sulphamic acid | 10.0 | 10.0 | 10.00 | 10.00 |
| PEG 20,000 | 4.0 | 4.0 | 4.00 | 4.00 |
| Sodium bicarbonate | 24.5 | 24.5 | 24.50 | 24.50 |
| Potassium persulfate | 15.0 | 15.0 | 15.00 | 15.00 |
| Sodium acid pyrophosphate | 7.0 | 7.0 | 7.00 | 7.00 |
| Pyrogenic silica | 2.0 | 2.0 | 2.00 | 2.00 |
| TAED* | 7.0 | 7.0 | 7.00 | 7.00 |
| Ricinoleylsulfosuccinate | 0.5 | 0.5 | 0.50 | 0.50 |
| Flavor | 1.0 | 1.0 | 1.00 | 1.00 |

| Alkaline Layer | | | | |
|---|---|---|---|---|
| Sodium perborate monohydrate | 32.0 | 32.0 | 32.00 | 32.00 |
| Sodium bicarbonate | 19.0 | 19.0 | 19.00 | 19.00 |
| EDTA | 3.0 | 3.0 | 3.00 | 3.00 |
| Sodium tripolyphosphate | 12.0 | 12.0 | 12.00 | 12.00 |
| PEG 20,000 | 2.0 | 2.0 | 2.00 | 2.00 |
| Potassium persulfate | 26.0 | 26.0 | 26.00 | 26.00 |
| Sodium carbonate | 2.0 | 2.0 | 2.00 | 2.00 |
| Pyrogenic silica | 2.0 | 2.0 | 2.00 | 2.00 |
| Dye/flavor | 2.0 | 2.0 | 2.00 | 2.00 |

| | | | | |
|---|---|---|---|---|
| *Tetraacetylethylene diamine | | | | |

In Examples 58-61, the BPN' variants recited in Table 2, among others, are substituted for Ala216Glu, with substantially similar results.

### 3. Contact Lens Cleaning Compositions

In another embodiment of the present invention, contact lens cleaning compositions comprise one or more enzyme variants of the present invention. Such contact lens cleaning compositions comprise an effective amount of one or more enzyme variants of the present invention, preferably from about 0.01 % to about 50% of one or more enzyme variants of the present invention, more preferably from about 0.01% to about 20%, more preferably still from about 1% to about 5%, by weight of the composition, and a contact lens cleaning carrier. Various contact lens cleaning composition formats such as tablets, liquids and the like are well known in the art (see for example U.S. Patent 4,863,627, Davies, Meaken and Rees, issued September 5, 1989; U.S. Patent Re. 32,672, Huth, Lam and Kirai, reissued May 24, 1988; U.S. Patent 4,609,493, Schäfer, issued September 2, 1986; U.S. Patent, 4,690,793, Ogunbiyi and Smith, issued September 1, 1987; U.S. Patent 4,614,549, Ogunbiyi, Riedhammer and Smith, issued September 30, 1986; and U.S. Patent 4,285,738, Ogata, issued August 25, 1981), and are generally appropriate for incorporation of one or more enzyme variants of the present invention for removing proteinaceous stains from contact lens.

The contact lens cleaning composition embodiment of the present invention is illustrated by the following examples.

**Examples 62-65**

| Enzymatic Contact Lens Cleaning Solution | | | | |
|---|---|---|---|---|
| | Example No. | | | |
| Component | 62 | 63 | 64 | 65 |
| Ile205Leu + Ala216Asp | 0.01 | 0.5 | 0.1 | 2.0 |
| Glucose | 50.00 | 50.0 | 50.0 | 50.0 |
| Nonionic surfactant (polyoxyethlenepolyoxypropylene copolymer) | 2.00 | 2.0 | 2.0 | 2.0 |
| Anionic surfactant (polyoxyethylenealkylphenylether sodium sulfricester) | 1.00 | 1.0 | 1.0 | 1.0 |
| Sodium chloride | 1.00 | 1.0 | 1.0 | 1.0 |
| Borax | 0.30 | 0.3 | 0.3 | 0.3 |
| Water | balance to 100% | | | |

In Examples 62-65, the BPN' variants recited in Table 2, among others, are substituted for Ile205Leu + Ala2l6Asp, with substantially similar results.

### SEQUENCE LISTING

(1) GENERAL INFORMATION:
   (i) APPLICANT:
   (ii) TITLE OF INVENTION: SERINE PROTEASE WITH DECREASED ADSORPTION AND INCREASED HYDROLYSIS
   (iii) NUMBER OF SEQUENCES: 1
   (iv) CORRESPONDENCE ADDRESS:
      (A) ADDRESSEE: THE PROCTER & GAMBLE COMPANY
      (B) STREET: 11810 EAST MIAMI RIVER ROAD
      (C) CITY: ROSS
      (D) STATE: OH
      (E) COUNTRY: USA
      (F) ZIP: 45061
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: CORSTANJE, BRAHM J.
      (B) REGISTRATION NUMBER: 34,804
   (ix) TELECOMMUNICATION INFORMATION:
      (A) TELEPHONE: 513-627-2858
      (B) TELEFAX: 513-627-0260
(2) INFORMATION FOR SEQ ID NO:1:
   (i) SEQUENCE CHARACTERISTICS;
      (A) LENGTH: 275 amino acids
      (B) TYPE: amino acid
      (D) TOPOLOGY: linear
   (ii) MOLECULE TYPE: protein
   (xi) SEQUENCE DESCRIPTION: SEQ ID NO:1:

## Claims

1. A BPN' variant comprising wild-type amino acid sequence, **characterized in that** the wild-type amino acid sequence comprises one of the following specific mutations, wherein the original amino acid occurring in wild-type is given first, the position number second, and the substituted amino acid third:
- **single mutation:**
Lys213Glu;
Ala216Glu;
Ala216Asp;
Ala216Gly;
Ser204Glu;
Val203Glu;
- **double mutation:**
Lys213Glu + Tyr217Leu;
Ile205Leu + Ala216Glu;
Ile205Leu + Ala216Asp;
Pro210Ala + Gly215Thr;
Lys213Glu + Ala216Glu;
Tyr214Phe + Tyr217Asn;
Gln206Glu + Ala216Glu;
Ala216Glu + Tyr217Leu;
Gln206Glu + Tyr217Leu;
Gln206Glu + Lys213Glu;
- **triple mutation:**
Gln206Pro + Gly211Ala + Ala216Glu;
Lys213Glu + Ala216glu + Tyr217Leu;
Ile205Val + Pro210Ala + Lys213Glu;
Gln206Glu + Ala216Glu + Tyr217Leu;
Gln206Glu + Lys213Glu + Tyr217Leu:
- **quadruple mutation:**
Pro210Ala + Lys213Glu + Ala216Glu + Tyr217 Leu;
Gln206Glu + Lys213Glu + Ala216Glu + Tyr217Leu;
Ser204Glu + Gln206Glu + Ala216Glu + Tyr217 Leu;
- **quintuple mutation:**
Ile205Leu + Pro210Ala + Lys213Glu + Ala216Glu + Tyr217Leu; and
Ser204Glu + Gln206Glu + Lys213Glu + Ala216Glu + Tyr217 Leu,
**characterized in that** the BPN' variants have decreased adsorption to and increased hydrolysis of, an insoluble substrate as compared to wild-type Subtilisin BPN'.

2. A cleaning composition selected from the group consisting of a hard surface cleaning composition, a dishwashing composition, an oral cleaning composition, a denture cleansing composition and a contact lens cleaning composition, **characterized in that** the cleaning composition comprises the BPN' variant of claim 1 and a hard surface cleaning carrier.

3. A hard surface cleaning composition comprising the BPN' variant of claim 1 and a hard surface cleaning carrier.

4. A fabric cleaning composition comprising a BPN' variant of claim 1.

## Patentansprüche

1. BPN'-Variante, die eine Wildtyp-Aminosäure-Sequenz umfasst, **dadurch gekennzeichnet, dass** die Wildtyp-Aminosäure-Sequenz eine der folgenden Mutationen umfasst, wobei die ursprüngliche Aminosäure, die im Wildtyp auftritt, als erstes, die Positionsnummer als zweites und die substituierte Aminosäure als drittes angegeben wird:
- **Einfache Mutation:**
Lys213Glu;
Ala216Glu;
Ala216Asp;
Ala216Gly;
Ser204Glu;
Val203Glu;
- **Zweifache Mutation:**
Lys213Glu + Tyr217Leu;
Ile205Leu + Ala216Glu;
Ile205Leu + Ala216Asp;
Pro210Ala + Gly215Thr;
Lys213Glu + Ala216Glu;
Tyr214Phe + Tyr217Asn;
Gln206Glu + Ala216Glu;
Ala216Glu + Tyr217Leu;
Gln206Glu + Tyr217Leu;
Gln206Glu + Lys213Glu;
- **Dreifache Mutation:**
Gln206Pro + Gly211Ala + Ala216Glu;
Lys213Glu + Ala216gGlu + Tyr217Leu;
Ile205Val + Pro210Ala + Lys213Glu;
Gln206Glu + Ala216Glu + Tyr217Leu;
Gln206Glu + Lys213Glu + Tyr217Leu;
- **Vierfache Mutation:**
Pro210Ala + Lys213Glu + Ala216Glu + Tyr217Leu;
Gln206Glu + Lys213Glu + Ala216Glu + Tyr217Leu;
Ser204Glu + Gln206Glu + Ala216Glu + Tyr217Leu;
- **Fünffache Mutation:**
Ile205Leu + Pro210Ala + Lys213Glu + Ala216Glu + Tyr217Leu; und
Ser204Glu + Gln206Glu + Lys213Glu + Ala216Glu + Tyr217Leu,
**dadurch gekennzeichnet, dass** die BPN'-Varianten im Vergleich zu Wildtyp-Subtilisin-BPN' verringerte Adsorption an und erhöhte Hydrolyse von einem unlöslichen Substrat aufweisen.

2. Reinigungszusammensetzung, ausgewählt aus der Gruppe, bestehend aus einer Reinigungszusammensetzung für harte Oberflächen, einer Geschirrspülzusammensetzung, einer Mundreinigungszusammensetzung, einer Zahnprothesenreinigungszusammensetzung und einer Kontaktlinsenreinigungszusammensetzung, **dadurch gekennzeichnet, dass** die Reinigungszusammensetzung die BPN'-Variante nach Anspruch 1 und einen Träger zur Hartoberflächenreinigung umfasst.

3. Reinigungszusammensetzung für harte Oberflächen, die die BPN'-Variante nach Anspruch 1 und einen Träger zur Hartoberflächenreinigung umfasst.

4. Stoffreinigungszusammensetzung, die eine BPN'-Variante nach Anspruch 1 umfasst.

## Revendications

1. Variant de BPN' comprenant une séquence aminoacide de type sauvage, **caractérisé en ce que** la séquence aminoacide de type sauvage comprend une des mutations spécifiques suivantes, dans laquelle l'acide aminé original se produisant sous un type sauvage est donné en premier, le numéro de position en deuxième, et l'acide aminé substitué en troisième :
- **mutation simple :**
Lys213Glu ;
Ala216Glu ;
Ala216Asp ;
Ala216Gly;
Ser204Glu ;
Val203Glu ;
- **mutation double :**
Lys213Glu + Tyr217Leu ;
Ile205Leu + Ala216Glu ;
Ile205Leu + Ala216Asp ;
Pro210Ala + Gly215Thr ;
Lys213Glu + Ala216Glu ;
Tyr214Phé + Tyr217Asn ;
Gln206Glu + Ala216Glu ;
Ala216Glu + Tyr217Leu ;
Gln206Glu + Tyr217Leu ;
Gln206Glu + Lys213Glu;
- **mutation triple :**
Gln206Pro + Gly211Ala + Ala216Glu ;
Lys213Glu + Ala216glu + Tyr217Leu ;
Ile205Val + Pro210Ala + Lys213Glu ;
Gln206Glu + Ala216Glu + Tyr217Leu ;
Gln206Glu + Lys213Glu + Tyr217Leu ;
- **mutation quadruple :**
Pro210Ala + Lys213Glu + Ala216Glu + Tyr217Leu ;
Gln206Glu + Lys213Glu + Ala216Glu + Tyr217Leu ;
Ser204Glu + Gln206Glu + Ala216Glu + Tyr217Leu ;
- **mutation quintuple :**
Ile205Leu + Pro210Ala + Lys213Glu + Ala216Glu + Tyr217Leu ; et
Ser204Glu + Gln206Glu + Lys213Glu + Ala216Glu + Tyr217Leu,
**caractérisé en ce que** les variants de BPN' ont diminué l'adsorption vers et augmenté l'hydrolyse d'un substrat insoluble par comparaison avec le BPN' de subtilisine de type sauvage.

2. Composition de nettoyage choisie parmi le groupe constitué d'une composition de nettoyage des surfaces dures, une composition de lavage de vaisselle, une composition de nettoyage oral, une composition de nettoyage de dentier et une composition de nettoyage de lentilles de contact, **caractérisée en ce que** la composition de nettoyage comprend le variant de BPN' selon la revendication 1 et un véhicule de nettoyage des surfaces dures.

3. Composition de nettoyage des surfaces dures comprenant le variant de BPN' selon la revendication 1 et un véhicule de nettoyage des surfaces dures.

4. Composition de nettoyage de tissus comprenant un variant de BPN' selon la revendication 1.
